# FASCICULE DE BREVET EUROPEEN

(11) **EP 0 738 710 B1**
(45) Date de publication et mention de la délivrance du brevet: **28.05.1997**
(21) Numéro de dépôt: 96400511.0
(22) Date de dépôt: 12.03.1996
(51) Int. Cl.: C07C 271/22, C07C 311/06, C07C 275/16

(54) **Dérivés d'ornithine, procédé de préparation, utilisation en cosmétique et compositions les comprenant**
Ornithinderivate, Verfahren zu ihrer Herstellung, ihre Verwendung in der Kosmetik und sie enthaltende Zusammensetzungen
Ornithine derivatives, process for their preparation, their use in cosmetics and compositions containing them

(30) Priorité: 20.04.1995 FR 9504746
(43) Date de publication de la demande: 23.10.1996
(73) Titulaire: L'OREAL, 75008 Paris (FR)
(72) Inventeur: Philippe, Michel, F-91320 Wissous (FR); Bordier, Thierry, F-93290 Tremblay en France (FR)
(74) Mandataire: Dodin, Catherine

(56) Documents cités:
- EP-A- 0 139 481
- EP-A- 0 336 265
- EP-A- 0 408 448
- EP-A- 0 447 287
- EP-A- 0 545 786

## Description

La présente invention a pour objet des composés, dérivés de l'ornithine, leur procédé de préparation, leur utilisation notamment en cosmétique et les compositions, notamment cosmétiques, les comprenant.

On connait des compositions, notamment cosmétiques, pharmaceutiques ou alimentaires, qui peuvent se présenter sous forme d'une poudre dite compacte. Il s'agit généralement de compositions anhydres pouvant être constituées principalement de particules solides et d'un liant gras, mises en forme par compression.
L'élaboration de telles compositions soulève toutefois de nombreuses difficultés car la composition finale doit être suffisamment homogène et compacte pour présenter une bonne aptitude au prélèvement et pour éviter par ailleurs une fragmentation pouvant être provoquée notamment par des chocs.
Il est décrit, dans la demande de brevet EP139481, des compositions cosmétiques utilisant comme agents pour modifier la surface de composés inorganiques, en vue d'en augmenter la dispersibilité, soit un dérivé monoacylé d'un acide aminé basique dont le groupement acyle aliphatique a 8-22 atomes de carbone, soit un dérivé N,N-diacylé d'un acide aminé basique dont les groupements acyles identiques ou différents, ont 1-22 atomes de carbone.
Il est également décrit, dans la demande de brevet EP336265, des compositions cosmétiques pour la mise en forme des cheveux comprenant comme agents tensioactifs, un dérivé N-monoacylé d'un acide aminé basique dont le groupement acyle a 8-22 atomes de carbone.
On constate toutefois que les dérivés acylés des acides aminés basiques décrits précédemment sont très difficilement compactables, voire intassables.

L'invention a pour but de proposer de nouveaux composés, qui permettent de faciliter l'obtention de telles compositions, tout en satisfaisant aux exigences précitées, sans présenter les inconvénients de l'art antérieur.

La présente invention a donc pour objet un composé de formule (I)

HOOC-CH(NH₂)-(CH₂)₃-NH-(X)ₕ-(CH₂)ⱼ-(CHOH)ₖ-(CH₂)ₘ-(O)ₙ-(CH₂)ₚ-R

dans laquelle
. R représente un radical alkyle hydrocarboné, linéaire ou ramifié, sature ou insaturé, ayant de 8 à 30 atomes de carbone, ou un radical alkyle perfluoré, linéaire ou ramifié, saturé ou insaturé, ayant 4 à 20 atomes de carbone,
. X est un radical divalent choisi parmi -CO-O-, -CO-NH- et -SO₂-,
. h, j, k, m et n sont, indépendamment, 0 ou 1, et p est 0 à 4, les sels des composés de formule (I), leurs isomères optiques de configuration D ou L, et leurs mélanges.

Un autre objet de l'invention est une composition, entre autre cosmétique, pharmaceutique, hygiénique ou alimentaire, comprenant au moins un composé de formule (I).
On a en effet constaté que les composés selon l'invention permettent de conférer à la composition cosmétique les comprenant des qualités d'étalement et d'adhésion à la peau particulièrement intéressantes, ainsi qu'un toucher onctueux et agréable, et une résistance à l'eau améliorée.

Encore un objet de l'invention est l'utilisation d'au moins un composé de formule (I) comme substance de revêtement de particules substrat.
On a en effet constaté que lesdites particules, généralement des poudres, lorsqu'elles étaient enrobées au moins partiellement par le composé selon l'invention, présentaient un toucher amélioré.

Enfin, un dernier objet de l'invention est l'utilisation d'au moins un composé de formule (I) pour améliorer le compactage de composition comprenant des poudres.

L'invention a donc pour premier objet un composé de formule (I):

HOOC-CH(NH₂)-(CH₂)₃-NH-(X)ₕ-(CH₂)ⱼ-(CHOH)ₖ-(CH₂)ₘ-(O)ₙ-(CH₂)ₚ-R

Parmi les composés selon l'invention, on peut citer N^{δ}-dodécyloxycarbonyl-L-ornithine, la N^{δ}-dodécanesulfonyl-L-ornithine et la N^{δ}-dodécylaminocarbonyl-L-ornithine.

Les sels des composés selon l'invention peuvent être choisis parmi les sels de cations inorganiques monovalents, tels que ceux de sodium, ou divalents tels que ceux de zinc ou de cuivre.
Les sels peuvent être aussi choisis parmi les sels de cations organiques tels que ceux d'aminopropanediol, de trishydroxyaminométhane, de glucamine et de N-méthylglucamine.

Les composés selon l'invention peuvent se présenter sous forme solide ayant une taille des particules comprise entre 10 - 500 000 nm, de préférence comprise entre 100 - 25000 nm.
Ces composés sont généralement insolubles dans les huiles et dans les solutions aqueuses dont le pH est compris entre 5 et 8.

La composition selon l'invention comprenant lesdits composés peut se présenter sous diverses formes telles que des dispersions, des lotions éventuellement épaissies ou gélifiées, des poudres éventuellement compactées, des laits, des crèmes, des sticks, des mousses ou des sprays lorsqu'elle est conditionnée en aérosol, des émulsions huile-dans-eau, eau-dans-huile ou multiple, de dispersions liposomiales ou encore de préparations solides.
Les composés selon l'invention peuvent être compris dans la composition en une proportion allant de 0,05% à 80% en poids, de préférence en une proportion de 0,5 à 30% en poids par rapport au poids total de la composition.
Les composés selon l'invention peuvent être présents dans la composition sous forme libre et/ou sous forme d'une association avec des particules substrats qu'ils enrobent au moins partiellement.

Outre le composé selon l'invention, la composition peut aussi comprendre au moins un additif choisi dans le groupe constitué par les agents tensioactifs, les corps gras, les solvants organiques, les silicones, les épaississants, les adoucissants, les filtres solaires, les agents de traitement, les agents anti-mousse, les agents hydratants, les parfums, les conservateurs, les agents antioxydants, les séquestrants, les agents de sapidité, les agents alcalinisants ou acidifiants, les charges et les pigments.
Parmi les corps gras utilisables dans la composition selon l'invention, on peut citer les huiles, les cires, les acides gras, les alcools gras et/ou leur mélange.
Les huiles peuvent être d'origine animale, végétale, minérale ou de synthèse. On peut citer en particulier l'huile de palme hydrogénée, l'huile de ricin hydrogénée, l'huile de vaseline, l'huile de paraffine, l'huile de purcellin. Les cires peuvent être d'origine animale, végétale, minérale ou de synthèse. On peut citer en particulier la cire d'abeille, la cire de montan, la cire de Carnauba, la cire de candelilla, la cire de canne à sucre, la cire du Japon, l'ozokérite, les cires microcristallines, la cire de paraffine, la cire de lanoline, la cire de lanoline hydrogénée et la cire de lanoline acétylée.

Parmi les particules pouvant être enrobées par les composés selon l'invention, on peut citer notamment les pigments, les charges particulaires et les microsphères telles que les microsphères creuses de copolymères de chlorure de vinylidène/acrylonitrile. Plus particulièrement, on peut citer, parmi les charges, les charges insolubles éventuellement colorées telles que des nanopigments d'oxydes métalliques comme les oxydes de titane, de zinc, de fer, de manganèse, de césium et/ou de zirconium.

La composition selon l'invention peut donc trouver une application en tant que compositions de maquillage telles que des fonds de teint, des crèmes teintées, des mascaras, des fards à joues et à paupières, des rouges à lèvres et des vernis à ongles.
Selon une forme particulière de réalisation, elle peut se présenter sous forme dite compacte, le composé selon l'invention facilitant le compactage des ingrédients desdites compositions.

Parmi ces compositions compactes, on peut citer notamment des fonds de teint, des fards à joues ou à paupières, et les rouges à lèvres.
La composition selon l'invention peut également se présenter sous forme d'une composition pharmaceutique ou hygiénique telles que des pâtes dentifrices, des compositions exfoliantes, des poudres pour le corps ou pour bébés et des poudres antitranspirantes, voire sous forme d'une composition alimentaire.

L'invention a également pour objet un procédé de préparation de composé de formule (I), consistant:
- à faire réagir en milieu aqueux et à pH basique, de l'ornithine ou l'un de ses sels, de configuration connue, avec une solution de sel de cuivre,
- à faire réagir sur la solution du complexe de cuivre un composé de formule (II) choisi parmi:

   R'-CO-O-(CH₂)ⱼ-(CHOH)ₖ-(CH₂)ₘ-(O)ₙ-(CH₂)ₚ-R (IIa)

   O=C=N-(CH₂)ⱼ-(CHOH)ₖ-(CH₂)ₘ-(O)ₙ-(CH₂)ₚ-R (IIb)

   R''-CO-NH-(CH₂)ⱼ-(CHOH)ₖ-(CH₂)ₘ-(O)ₙ-(CH₂)ₚ-R (IIc)

   Cl-SO₂-(CH₂)ⱼ-(CHOH)ₖ-(CH₂)ₘ-(O)ₙ-(CH₂)ₚ-R (IId)

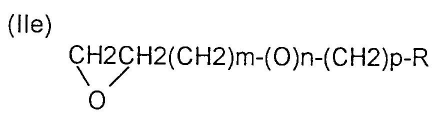

   dans laquelle R' représente un atome de chlore, un radical chlorométhyle ou un radical azolyle et R'' représente un radical azolyle,
- à traiter le sel de cuivre d'ornithine substituée obtenu par un agent décomplexant et, éventuellement
- à purifier le composé obtenu.

Parmi les solutions de sels de cuivre utilisées dans le procédé selon l'invention, on peut citer en particulier les solutions de sulfate de cuivre.
Le pH basique du milieu réactionnel, est de préférence compris entre 8 et 14.
Le radical azolyle peut être un radical imidazolyle.
Le composé de formule (II) peut être additionné avec ou sans solvant.
L'agent décomplexant utilisé peut être une solution aqueuse du sel disodique de l'acide EDTA ou d'un acide tel que l'acide chlorhydrique.

Des exemples de préparation de composés selon l'invention sont donnés ci-après, ainsi qu'un exemple de composition comprenant un tel composé.

### EXEMPLE 1: Préparation de la N^{δ}-dodécyloxycarbonyl-L-ornithine (R = C₁₂H₂₅, X = -COO-, h = 1, j = k = m= n = p = 0)

Dans un ballon de 100 ml, 5 g de L-ornithine monochlorhydrate sont mis en solution dans 24 ml d'une solution aqueuse de soude à 10%. Une solution de 3,7 g de sulfate de cuivre pentahydraté dans 40 ml d'eau est ajoutée. Au mélange réactionnel refroidi à 5°C, on ajoute 2,49 g d'hydrogénocarbonate de sodium, puis 1 éq. de chloroformiate de dodécyle en solution dans le THF.
Après 16 heures sous agitation à température ambiante, le milieu réactionnel est filtré et le précipité est lavé et séché sous pression réduite. Le complexe est traité au reflux pendant 2 h par une solution aqueuse de sel disodique dihydraté de l'acide EDTA à 10%.
On obtient 7,4 g (rendement 73%) de produit blanc.

L'analyse chimique donne les caractéristiques suivantes :
- Fusion : T> 260°C
- Spectre de masse : m/z 345,2 (MH+); 299,2; 282,2

| - Analyse élémentaire :(C₁₈H₃₆N₂O₄, poids moléculaire 344,499) | | | | |
|---|---|---|---|---|
| | C | H | F | N |
| % calculé | 62,76 | 10,53 | 8,13 | 18,58 |
| % mesuré | 62,61 | 10,60 | 8,37 | 18,52 |

- Granulométrie (coulter counter TA2): en nombre (moyenne) 3,15 µm
- Chromatographie sur couche mince : HPCCM (silice Merck 60F254), avec comme éluant, un mélange NH₄OH 6 / CH₃OH 47 / CH₂Cl₂ 47 On obtient un rapport frontal Rf = 0,59.

### EXEMPLE 2 : Préparation de la N^{δ}-dodécanesulfonyl-L-ornithine (R = C₁₂H₂₅, X = -SO₂-, h = 1, j = k = m= n = p = 0)

Dans un ballon de 100 ml, 5 g de L-ornithine monochlorhydrate sont mis en solution dans 24 ml d'une solution aqueuse de soude à 10%. Une solution de 3,7 g de sulfate de cuivre pentahydraté dans 40 ml d'eau est ajoutée. Au mélange réactionnel refroidi à 5°C, on ajoute 2,49 g d'hydrogénocarbonate de sodium, puis 1 éq. de chlorure de dodécanesulfonyle en solution dans le THF. Après 16 heures sous agitation à température ambiante, le milieu réactionnel est filtré et le précipité est lavé et séché sous pression réduite. Le complexe est traité au reflux pendant 2 h par une solution aqueuse de sel disodique dihydraté de l'acide EDTA à 10%.
On obtient 3,9 g (rendement 36%) de produit blanc.

L'analyse chimique donne les caractéristiques suivantes :
- Fusion : T> 260°C
- Spectre de masse: m/z 365 (MH+); 347,2; 302,2

| - Analyse élémentaire :(C₁₇H₃₆N₂O₄S, poids moléculaire 364,551) | | | | | |
|---|---|---|---|---|---|
| | C | H | N | O | S |
| % calculé | 56,01 | 9,95 | 7,68 | 17,56 | 8,80 |
| % mesuré | 56,07 | 10,16 | 7,76 | 17,64 | 8,93 |

- Granulométrie (coulter counter TA2) : en nombre (moyenne) 2,19 µm
- Chromatographie sur couche mince : HPCCM (silice Merck 60F254), avec comme éluant, un mélange NH₄OH 6 / CH₃OH 47 / CH₂Cl₂ 47 On obtient un rapport frontal Rf = 0,48.

### EXEMPLE 3 : Préparation d'une poudre compactée

On prépare une poudre ayant la composition suivante :

| | |
|---|---|
| - Talc | 38,4 g |
| - Oxychlorure de bismuth | 10 g |
| - Stéarate de zinc | 4 g |
| - Composé de l'exemple 1 | 20 g |
| - Poudre de Nylon | 20 g |
| - Oxydes de fer | 1,6 g |
| - Huile de vaseline | 6 g |

La poudre est obtenue de la façon suivante : on broie les constituants, sauf l'huile de vaseline, dans un broyeur de type KENWOOD pendant environ 5 minutes sous faible agitation. On ajoute l'huile de vaseline et on broie l'ensemble environ 2 minutes à la même vitesse, puis 3 minutes à une vitesse plus rapide. On tamise ensuite la préparation sur un tamis de 0,16 mm, puis on compacte ce mélange dans des coupelles (40 bars).
On obtient une poudre compactée présentant une bonne adhésion, qui s'étale bien et de manière agréable sur la peau, tout en étant douce au toucher.

### EXEMPLE 4 : Préparation d'une poudre compactée

On prépare une poudre ayant la composition suivante :

| | |
|---|---|
| - Talc | 38,4 g |
| - Oxychlorure de bismuth | 10 g |
| - Stéarate de zinc | 4 g |
| - Composé de l'exemple 2 | 20 g |
| - Poudre de Nylon | 20 g |
| - Oxydes de fer | 1,6 g |
| - Huile de vaseline | 6 g |

La poudre est obtenue de façon similaire à l'exemple 3.
On obtient une poudre compactée présentant une bonne adhésion, qui s'étale bien et de manière agréable sur la peau, tout en étant douce au toucher.

### EXEMPLE 5

On compare les compositions des exemples 3 et 4 avec :
. la même composition dans laquelle les composés de l'invention sont remplacés totalement par du mica (composition témoin I) et
. la même composition dans laquelle les composés de l'invention sont remplacés totalement par de l'Amihope vendu par Ajinomoto (composition témoin II).

On effectue ensuite un test de chute qui consiste à laisser tomber, à deux reprises, d'une hauteur de 1 m, les compacts obtenus, puis à les peser de manière à déterminer le % de poudre éliminée.

On obtient les résultats suivants :

| | témoin I | témoin II | exemple 3 | exemple 4 |
|---|---|---|---|---|
| perte (% en poids) | 3% | 1% | 0,5% | 0% |
| commentaires | bon délitage | délitage moyen | délitage moyen/faible | presque pas de délitage |

On constate donc que les compositions témoins s'effritent beaucoup plus facilement que les compositions selon l'invention.
La présence des composés selon l'invention permet d'améliorer nettement la cohésion du produit compacté.

## Revendications

1. Composé, dérivé d'ornithine, de formule (I) :
HOOC-CH(NH₂)-(CH₂)₃-NH-(X)ₕ-(CH₂)ⱼ-(CHOH)ₖ-(CH₂)ₘ-(O)ₙ-(CH₂)ₚ-R
dans laquelle
. R représente un radical alkyle hydrocarboné, linéaire ou ramifié, sature ou insaturé, ayant de 8 à 30 atomes de carbone, ou un radical alkyle perfluoré, linéaire ou ramifié, saturé ou insaturé, ayant 4 à 20 atomes de carbone,
. X est un radical divalent choisi parmi -CO-O-, -CO-NH- et -SO₂-,
. h, j, k, m et n sont, indépendamment, 0 ou 1, et
. p est 0 à 4,
les sels des composés de formule (I), leurs isomères optiques de configuration D ou L, et leurs mélanges.

2. Composé selon la revendication 1, dans lequel les sels sont choisis parmi les sels de cations inorganiques monovalents ou divalents, et les sels de cations organiques.

3. Composé selon l'une des revendications précédentes, choisi parmi la N^{δ}-dodécyloxycarbonyl-L-ornithine, la N^{δ}-dodécanesulfonyl-L-ornithine et la N^{δ}-dodécylaminocarbonyl-L-ornithine.

4. Composé selon l'une des revendications précédentes, ayant une taille de particules comprise entre 10 nm et 500 000 nm.

5. Composition caractérisée en ce qu'elle comprend au moins un composé selon l'une des revendications 1 à 4.

6. Composition selon la revendication 5, dans laquelle le composé de formule (I) est compris entre 0,05 et 80 % en poids par rapport au poids total de la composition.

7. Composition selon la revendication 6, dans laquelle le composé de formule (I) est compris entre 0,5 et 30 % en poids par rapport au poids total de la composition.

8. Composition selon l'une des revendications 5 à 7, dans laquelle le composé de formule (I) est présent sous forme libre et/ou sous forme d'une association avec des particules substrats qu'il enrobe au moins partiellement.

9. Composition selon la revendication 8, dans laquelle les particules substrats sont choisies parmi les pigments, les charges particulaires et les microsphères telles que les microsphères creuses de copolymères de chlorure de vinylidène/acrylonitrile.

10. Composition selon l'une des revendications 5 à 9, se présentant sous forme d'une composition cosmétique, pharmaceutique, hygiénique ou alimentaire.

11. Composition selon l'une des revendications 5 à 10, se présentant sous forme de dispersions, de lotions éventuellement épaissies ou gélifiées, de poudres éventuellement compactées, de laits, de crèmes, de sticks, de mousses, de sprays, d'émulsions, de dispersions liposomiales ou de préparations solides.

12. Composition selon l'une des revendications 5 à 11, se présentant sous forme d'une composition de maquillage telle que fond de teint, crème teintée, mascara, fard à joues et à paupières, rouge à lèvres, vernis à ongles, ou sous forme d'une composition hygiénique telle que composition exfoliante, pâte dentifrice, poudre pour le corps ou pour bébés et/ou poudre antitranspirante.

13. Procédé de préparation d'un composé selon l'une des revendications 1 à 4, consistant à faire réagir en milieu aqueux et à pH basique, de l'ornithine ou l'un de ses sels, de configuration connue, avec une solution de sel de cuivre; puis à faire réagir sur la solution du complexe de cuivre un composé de formule (II) choisi parmi :
R'-CO-O-(CH₂)ⱼ-(CHOH)ₖ-(CH₂)ₘ-(O)ₙ-(CH₂)ₚ-R (IIa)
O=C=N-(CH₂)ⱼ-(CHOH)ₖ-(CH₂)ₘ-(O)ₙ-(CH₂)ₚ-R (IIb)
R''-CO-NH-(CH₂)ⱼ-(CHOH)ₖ-(CH₂)ₘ-(O)ₙ-(CH₂)ₚ-R (IIc)
Cl-SO₂-(CH₂)ⱼ-(CHOH)ₖ-(CH₂)ₘ-(O)ₙ-(CH₂)ₚ-R (IId) 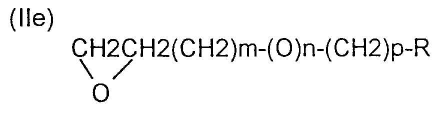
dans laquelle R' représente un atome de chlore, un radical chlorométhyle ou un radical azolyle et R'' représente un radical azolyle; et à traiter le sel de cuivre d'ornithine substituée obtenu par un agent décomplexant.

14. Utilisation d'au moins un composé selon l'une des revendication 1 à 4, en tant que substance de revêtement de particules substrat.

15. Utilisation d'au moins un composé selon l'une des revendication 1 à 4, pour améliorer le compactage de composition comprenant des poudres.

## Claims

1. Compound, which is derived from ornithine, of formula (I):
HOOC-CH(NH₂)-(CH₂)₃-NH-(X)ₕ-(CH₂)ⱼ-(CHOH)ₖ-(CH₂)ₘ-(O)ₙ-(CH₂)ₚ-R
in which
• R represents a saturated or unsaturated, linear or branched, hydrocarbon alkyl radical having from 8 to 30 carbon atoms or a saturated or unsaturated, linear or branched, perfluorinated alkyl radical having 4 to 20 carbon atoms,
• X is a divalent radical chosen from -CO-O-, -CO-NH- and -SO₂-,
• h, j, k, m and n are, independently, 0 or 1 and
• p is 0 to 4,
the salts of the compounds of formula (I), their optical isomers of D or L configuration, and their mixtures.

2. Compound according to Claim 1, in which the salts are chosen from the salts of monovalent or divalent inorganic cations and the salts of organic cations.

3. Compound according to one of the preceding claims, chosen from N^{δ}-dodecyloxycarbonyl-L-ornithine, N^{δ}-dodecylsulphonyl-L-ornithine and N^{δ}-dodecylaminocarbonyl-L-ornithine.

4. Compound according to one of the preceding claims, having a particle size of between 10 nm and 500,000 nm.

5. Composition, characterized in that it comprises at least one compound according to one of Claims 1 to 4.

6. Composition according to Claim 5, in which the compound of formula (I) is between 0.05 and 80 % by weight with respect to the total weight of the composition.

7. Composition according to Claim 6, in which the compound of formula (I) is between 0.5 and 30 % by weight with respect to the total weight of the composition.

8. Composition according to one of Claims 5 to 7, in which the compound of formula (I) is present in the free form and/or in the form of a combination with substrate particles which it at least partially coats.

9. Composition according to Claim 8, in which the substrate particles are chosen from pigments, particulate fillers and microspheres such as hollow vinylidene chloride/acrylonitrile copolymer microspheres.

10. Composition according to one of Claims 5 to 9, which is provided in the form of a cosmetic, pharmaceutical, hygiene or food composition.

11. Composition according to one of Claims 5 to 10, which is provided in the form of dispersions, optionally thickened or gelled lotions, optionally compacted powders, milks, creams, sticks, foams, sprays, emulsions, liposomal dispersions or solid preparations.

12. Composition according to one of Claims 5 to 11, which is provided in the form of a make-up composition such as a foundation, tinted cream, mascara, blusher, eye shadow, lipstick or nail varnish or in the form of a hygiene composition such as an exfoliative composition, toothpaste, powder for the body or for babies, and/or anti-perspirant powder.

13. Process for the preparation of a compound according to one of Claims 1 to 4, which consists in reacting, in aqueous medium and at basic pH, ornithine or one of its salts, of known configuration, with a solution of a copper salt; and then in reacting the solution of the copper complex with a compound of formula (II) chosen from:
R'-CO-O-(CH₂)ⱼ-(CHOH)ₖ-(CH₂)ₘ-(O)ₙ-(CH₂)ₚ-R (IIa)
O=C=N-(CH₂)ⱼ-(CHOH)ₖ-(CH₂)ₘ-(O)ₙ-(CH₂)ₚ-R (IIb)
R''-CO-NH-(CH₂)ⱼ-(CHOH)ₖ-(CH₂)ₘ-(O)ₙ-(CH₂)ₚ-R (IIc)
Cl-SO₂-(CH₂)ⱼ-(CHOH)ₖ-(CH₂)ₘ-(O)ₙ-(CH₂)ₚ-R (IId) 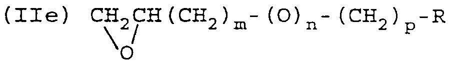
in which R' represents a chlorine atom, a chloromethyl radical or an azolyl radical and R'' represents an azolyl radical; and in treating the copper salt of the substituted ornithine obtained with a decomplexing agent.

14. Use of at least one compound according to one of Claims 1 to 4, as a substance for coating substrate particles.

15. Use of at least one compound according to one of Claims 1 to 4, for improving the compacting of a composition comprising powders.

## Patentansprüche

1. Verbindung, die von Ornithin abstammt, der Formel (I)
HOOC-CH(NH₂)-(CH₂)₃-NH-(X)ₕ-(CH₂)ⱼ-(CHOH)ₖ-(CH₂)ₘ-(O)ₙ-(CH₂)ₚ-R,
in der bedeuten:
- R eine geradkettige oder verzweigte, gesättigte oder ungesättigte Kohlenwasserstoffalkylgruppe mit 8 bis 30 Kohlenstoffatomen oder eine perfluorierte, geradkettige oder verzweigte, gesättigte oder ungesättigte Alkylgruppe mit 4 bis 20 Kohlenstoffatomen,
- X eine zweiwertige Gruppe, die unter -CO-O-, -CO-NH- und -SO₂- ausgewählt ist,
- h, j, k, m und n unabhängig 0 oder 1
- p 0 bis 4,
sowie die Salze der Verbindungen der Formel (I), ihre optischen Isomere mit D- oder L-Konfiguration und ihre Gemische.

2. Verbindung nach Anspruch 1, wobei die Salze unter den Salzen anorganischer einwertiger oder zweiwertiger Kationen und den Salzen organischer Kationen ausgewählt sind.

3. Verbindung nach einem der vorhergehenden Ansprüche, die unter N^{δ}-Dodecyloxycarbonyl-L-ornithin, N^{δ}-Dodecansulfonyl-L-ornithin und N^{δ}-Dodecylaminocarbonyl-L-ornithin ausgewählt ist.

4. Verbindung nach einem der vorhergehenden Ansprüche, die eine Partikelgröße von 10 nm bis 500 000 nm aufweist.

5. Zusammensetzung, dadurch gekennzeichnet, daß sie mindestens eine Verbindung nach einem der Ansprüche 1 bis 4 enthält.

6. Zusammensetzung nach Anspruch 5, wobei die Verbindung der Formel (I) in einem Anteil von 0,05 bis 80 Gew.-%, bezogen auf das Gesamtgewicht der Zusammensetzung, enthalten ist.

7. Zusammensetzung nach Anspruch 6, wobei die Verbindung der Formel (I) in einem Anteil von 0,5 bis 30 Gew.-%, bezogen auf das Gesamtgewicht der Zusammensetzung, enthalten ist.

8. Zusammensetzung nach einem der Ansprüche 5 bis 7, wobei die Verbindung der Formel (I) in freier Form oder in Form einer Kombination mit Substratpartikeln, die sie zumindest teilweise umhüllt, vorhanden ist.

9. Zusammensetzung nach Anspruch 8, wobei die Substratpartikel unter Pigmenten, teilchenförmigen Füllstoffen und Mikrokügelchen, wie z.B. hohlen Mikrokügelchen aus Vinylidenchlorid/Acrylnitril-Copolymeren, ausgewählt sind.

10. Zusammensetzung nach einem der Ansprüche 5 bis 9, die in Form einer kosmetischen Zusammensetzung, pharmazeutischen Zusammensetzung, Hygienezusammensetzung oder Lebensmittelzusammensetzung vorliegt.

11. Zusammensetzung nach einem der Ansprüche 5 bis 10, die in Form von Dispersionen, Lotionen, die ggf. verdickt oder geliert sind, ggf. verdichteten Pulvern bzw. gepreßten Pudern, Milchen, Cremes, Stiften, Schäumen, Sprays, Emulsionen, liposomalen Dispersionen oder festen Zubereitungen vorliegt.

12. Zusammensetzung nach einem der Ansprüche 5 bis 11, die in Form einer Schminkzusammensetzung, wie z.B. Make-up, einer getönten Creme, Wimperntusche, Rouge und Lidschatten, Lippenstift, Nagellack oder in Form einer Hygienezusammensetzung, wie z.B. einer abradierenden Zusammensetzung, Zahncreme, Körperpuder oder Puder für Babys und/oder schweißverhütenden Pulver vorliegt.

13. Verfahren zur Herstellung einer Verbindungen nach einem der Ansprüche 1 bis 4, das darin besteht, in wäßrigem Medium und bei basischem pH-Wert Ornithin oder eines seiner Salze mit bekannter Konfiguration mit einer Lösung eines Kupfersalzes reagieren zu lassen, mit der Lösung des Kupferkomplexes eine Verbindung der Formel (II), die ausgewählt wird unter
R'-CO-O-(CH₂)ⱼ-(CHOH)ₖ-(CH₂)ₘ-(O)ₙ-(CH₂)ₚ-R (IIa)
O=C=N-(CH₂)ⱼ-(CHOH)ₖ-(CH₂)ₘ-(O)ₙ-(CH₂)ₚ-R (IIb)
R''-CO-NH-(CH₂)ⱼ-(CHOH)ₖ-(CH₂)ₘ-(O)ₙ-(CH₂)ₚ-R (IIc)
Cl-SO₂-(CH₂)ⱼ-(CHOH)ₖ-(CH₂)ₘ-(O)ₙ-(CH₂)ₚ-R (IId)
und 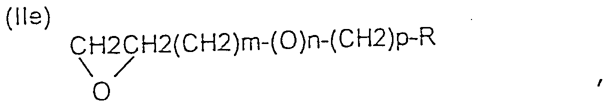 worin R' ein Chloratom, eine Chlormethylgruppe oder eine Azolylgruppe darstellt und R'' eine Azolylgruppe bedeutet, reagieren zu lassen und das erhaltene Kupfersalz des substituierten Ornithins mit einem Dekomplexierungsmittel zu behandeln.

14. Verwendung mindestens einer Verbindung nach einem der Ansprüche 1 bis 4 als Substanz zur Umhüllung von Substratpartikeln.

15. Verwendung mindestens einer Verbindung nach einem der Ansprüche 1 bis 4 zur Verbesserung der Verdichtung von Zusammensetzungen, die Pulver enthalten.
